# EUROPEAN PATENT APPLICATION

(11) **EP 4 737 885 A1**
(43) Date of publication of application: **06.05.2026**
(21) Application number: 24832158.0
(22) Date of filing: 01.07.2024
(51) Int. Cl.: G01N 21/64

(54) **DETECTING KIT, DETECTING DEVICE, AND DETECTING METHOD FOR DETECTING TRACE AMOUNT OF HEAVY METAL IN SAMPLE**

(30) Priority: 30.06.2023 US 202363524417 P
(71) Applicant: Aoyagi, Tetsuji, Yokohama-shi, Kanagawa 227-0062 (JP)
(72) Inventor: TAKAMURA, Takeji, Atsugi-shi Kanagawa 243-0011 (JP); OTA, Miki, Ayase-shi Kanagawa 252-1126 (JP); AOYAGI, Tetsuji, Yokohama-shi, Kanagawa 227-0062 (JP)
(74) Representative: Uexküll & Stolberg
(86) International application number: PCT/JP2024/023840
(87) International publication number: WO 2025/005303

(57) **Abstract**

An object is to provide a detection kit and a detection device for detecting a trace amount of a heavy metal contained in water, soil, a food ingredient, a food product or the like rapidly, highly sensitively, and selectively by a simple method in or around a spot such as a water source, a farmland, or a food processing plant, and a detection method using these. The present invention provides a detection kit, for detecting a trace amount of a heavy metal in a sample, including a DNA aptamer labeled with a fluorescent molecule, and specifically binding to a heavy metal. The heavy metal contained in the sample may be selected from cadmium, mercury, and arsenic. The detection may be performed by visual observation, fluorescence analysis, or fluorescence polarization analysis.

## Description

### [Technical Field]

The present invention relates to a detection kit, a detection device, and a detection method for detecting a trace amount of a heavy metal in a sample.

### [Background Art]

It has been conventionally known that problems are caused by heavy metals contained in water and soil at construction sites and farmlands. For example, when a heavy metal is present in water or soil of rice paddies or vegetable farms, the heavy metal accumulates in the cultivated crops, such as rice and vegetables. By ingesting these crops, or ingesting heavy-metal-containing groundwater as drinking water, the heavy metal is taken into human body. Similar problems also arise due to pesticides and impurities contained in food ingredients and food products.

When the ingested heavy metal is toxic, serious adverse effects are caused on health. Heavy metals such as cadmium, mercury, and arsenic can cause acute or chronic symptoms in the human body, including developmental disorders in children, issues during pregnancy, damage to the liver, kidneys, intestines and the like, anemia, and the onset of cancer. Cadmium, mercury, and arsenic are consistently among the top ten chemical substances of major public health concern listed by the World Health Organization. Moreover, cadmium and related compounds thereof are currently listed in Group A of the IARC classification as causes of human cancer.

Many conventional methods for testing heavy metals in water and soil involve time-consuming detection, or use expensive equipment that must be handled carefully for detecting heavy metals. PTL 1 discloses a hazardous substance content analysis method for measuring the content of hazardous substances, such as heavy metals eluted from soil, using an X-ray fluorescence analyzer. PTL 2 discloses a method in which a chelate complex is created by adding a chelating agent to a test solution obtained from soil, and is subjected to X-ray fluorescence analysis.

Although cadmium can be detected by several instrumental analysis techniques using an atomic absorption spectrometry and an inductively coupled plasma device, these techniques are known to have relatively high initial and running costs (NPLs 1 and 2). In addition, many conventional detection methods require large, expensive equipment and trained operators.

### [Citation List]

### [Patent Literature]

[PTL 1] the Publication of Japanese Patent No. 4647405
[PTL 2] Japanese Unexamined Patent Application, Publication No. 2004-294329

### [Non Patent Literature]

[NPL 1] Chen K, Mou P, Zhu A, Chen P, Chen J, Gao G, Wang X, Feng X, Yu C (2023) Environmental Monitoring and Assessment 195.
[NPL 2] Pyle SM, Nocerino JM, Deming SN, Palasota JA, Palasota JM, Miller EL, Hillman DC, Kuharic CA, Cole WH, Fitzpatrick PM, Watson MA, Nichols KD (1996) Environmental Science & Technology 30:204-213.

### [Summary of Invention]

### [Technical Problem]

Considering the above-described problems, if the presence of heavy metals in water or soil can be rapidly ascertained by a simple method near water sources, farmlands, or food processing plants, measures, such as quickly cleaning contaminated water or soil, or halting product shipment, can be taken. For example, a simple method for detecting a lower concentration (lower than 10⁻¹⁰ mol/L) of a heavy metal in water is desired, but has not yet been achieved. There is a strong demand for a system for rapidly, inexpensively, and simply detecting a trace amount of a heavy metal in an environment such as water or soil.

The present invention has been made in view of these circumstances, and an object is to provide a detection kit, a detection device, and a detection method for highly sensitively and rapidly detecting, near water sources, farmlands, or food processing plants, a trace amount of a heavy metal in water, soil, food ingredients, and food products by a simple technique.

### [Solution to Problem]

In order to solve the aforementioned problems, a detection kit, a detection device, and a detection method for detecting a trace amount of a heavy metal in a sample of the present invention employ the following solutions:

A first aspect of the present invention provides a detection kit for detecting a trace amount of a heavy metal in a sample, including a DNA aptamer that is labeled with a fluorescent molecule, and selectively binds to a specific heavy metal.

In the first aspect, the heavy metal may be at least one selected from the group consisting of cadmium, mercury, and arsenic.

In the first aspect, detection may be performed by visual observation, fluorescence analysis, or fluorescence polarization analysis.

In the first aspect, the sample may be water, soil, a food ingredient, or a food product.

In the first aspect, the DNA aptamer may be labeled with two fluorescent molecules.

The first aspect may further include MOPS buffer, HEPES buffer, or a carbonate-bicarbonate buffer.

A second aspect of the present invention provides a detection device including: a DNA aptamer that is labeled with a fluorescent molecule, and selectively binds to a specific heavy metal; and a fluorescence measurement unit for measuring the fluorescence intensity of the fluorescent molecule of the DNA aptamer, wherein a trace amount of the heavy metal in a sample is detected by measuring fluorescence intensity or fluorescence polarization intensity derived from the fluorescent molecule labeling the DNA aptamer bound to the specific heavy metal.

In the second aspect, the heavy metal may be at least one selected from the group consisting of cadmium, mercury, and arsenic.

In the second aspect, detection of the heavy metal may be performed by fluorescence analysis or fluorescence polarization analysis.

In the second aspect, the sample may be water, soil, a food ingredient, or a food product.

A third aspect of the present invention provides a detection method for detecting a trace amount of a heavy metal in a sample, including the steps of: obtaining a mixed solution by mixing a solution, in which a DNA aptamer labeled with a fluorescent molecule and specifically binding to the specific heavy metal is dissolved, with a liquid containing a sample potentially containing the specific heavy metal; measuring fluorescence intensity or fluorescence polarization intensity derived from the fluorescent molecule of the DNA aptamer contained in the mixed solution; and detecting the heavy metal based on the result of the measurement.

In the third aspect, the step of measuring fluorescence polarization intensity may further include a step of analyzing a concentration of the heavy metal.

### [Advantageous Effects of Invention]

When the detection kit and the detection method of the present invention are employed, it is possible to rapidly and simply detect a trace amount of a heavy metal in the sub-nanomolar order without the need for highly advanced detection technology. Since a simply usable detection device is provided, it is possible to install the detection device of the present invention near a spot where water quality testing is necessary, such as a farm, a well, a water purification plant, a poultry farm, or a food processing factor, for easily detecting the presence or absence of a heavy metal in a sample in a short time after sample collection.

### [Brief Description of Drawings]

[Fig. 1A]

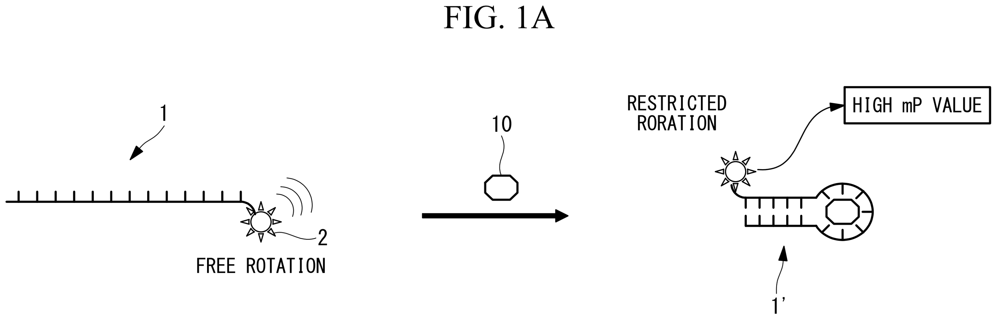

   Fig. 1A is a conceptual diagram illustrating a method for detecting a trace amount of a heavy metal through fluorescence polarization using a DNA aptamer of the present invention.
[Fig. 1B]

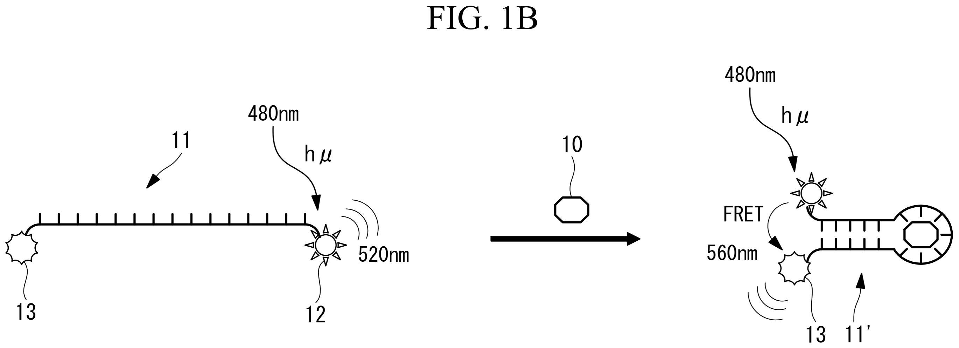

   Fig. 1B is a conceptual diagram illustrating a method for detecting a trace amount of a heavy metal utilizing FRET using a DNA aptamer having a FITC group at one end and TAMRA group at the other end.
[Fig. 2]

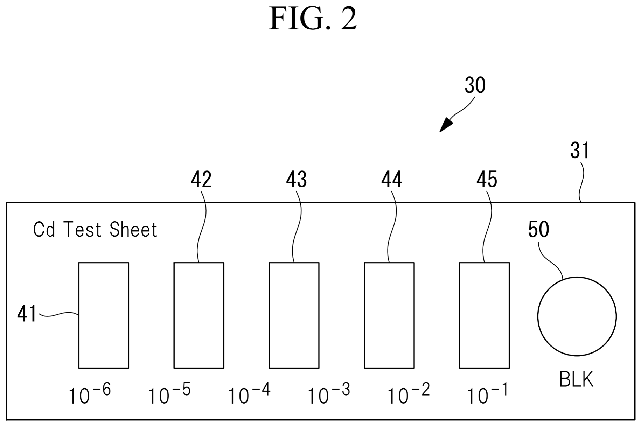

   Fig. 2 is a diagram illustrating an example of a heavy metal detection member used in the method for detecting a heavy metal of the present invention.
[Fig. 3]

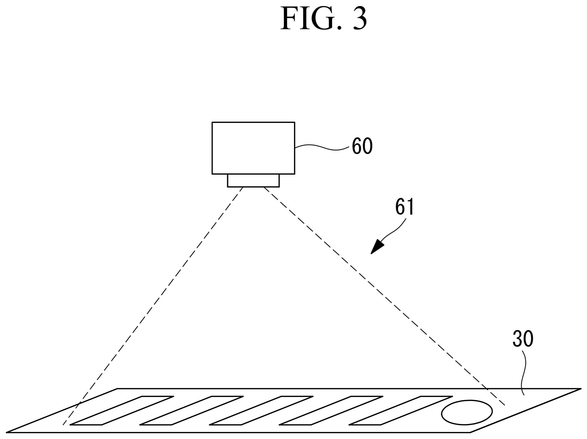

   Fig. 3 is an image diagram of heavy metal detection using the heavy metal detection member of the present invention.
[Fig. 4]

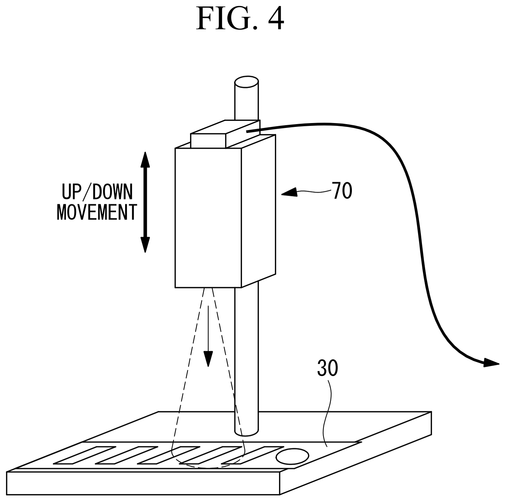

   Fig. 4 is a diagram illustrating an example of heavy metal detection device used in the method for detecting a heavy metal of the present invention.
[Fig. 5]

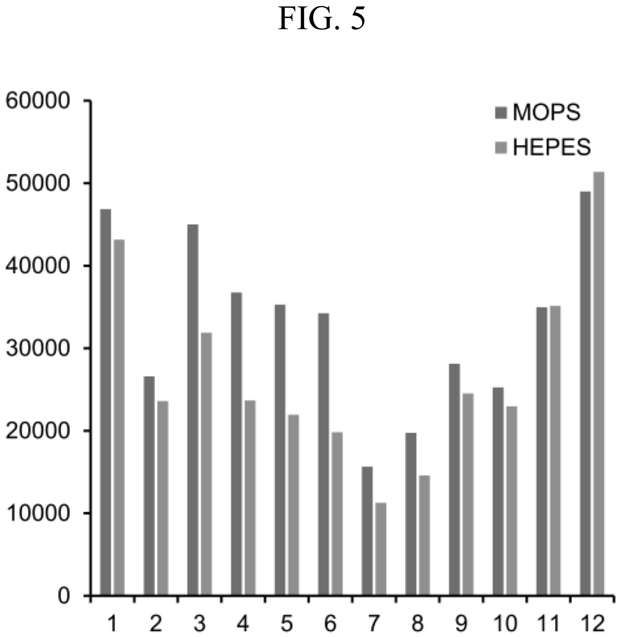

   Fig. 5 is a graph comparing the fluorescence intensity of aptamers 1 to 12 dissolved in MOPS buffer and HEPES buffer. For each aptamer, the left bar shows the result obtained using MOPS buffer, and the right bar shows the result obtained using HEPES buffer.
[Fig. 6]

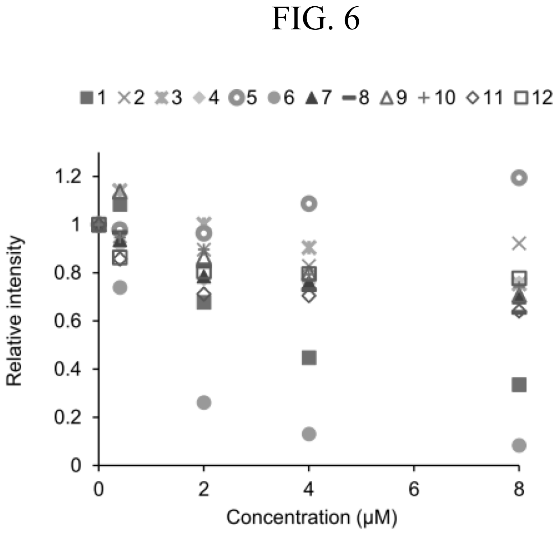

   Fig. 6 is a graph illustrating the relationship between the final cadmium concentration in solutions obtained by mixing the aptamers 1 to 12 with a cadmium solution, and the fluorescence intensity of the mixed solutions.
[Fig. 7]

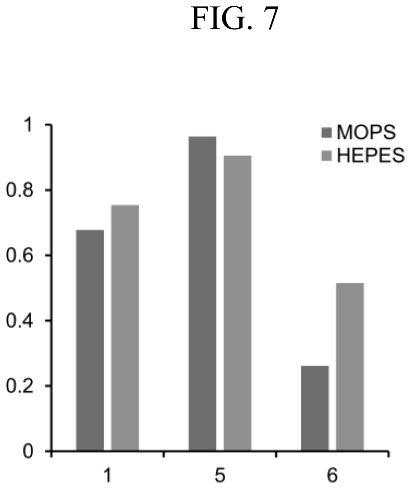

   Fig. 7 is a graph illustrating buffer dependence of the fluorescence intensity in mixed solutions of the aptamers 1, 5, and 6 dissolved in MOPS buffer or HEPES buffer, with a cadmium solution. For each aptamer, the left bar shows the result obtained using MOPS buffer, and the right bar shows the result obtained using HEPES buffer.
[Fig. 8]

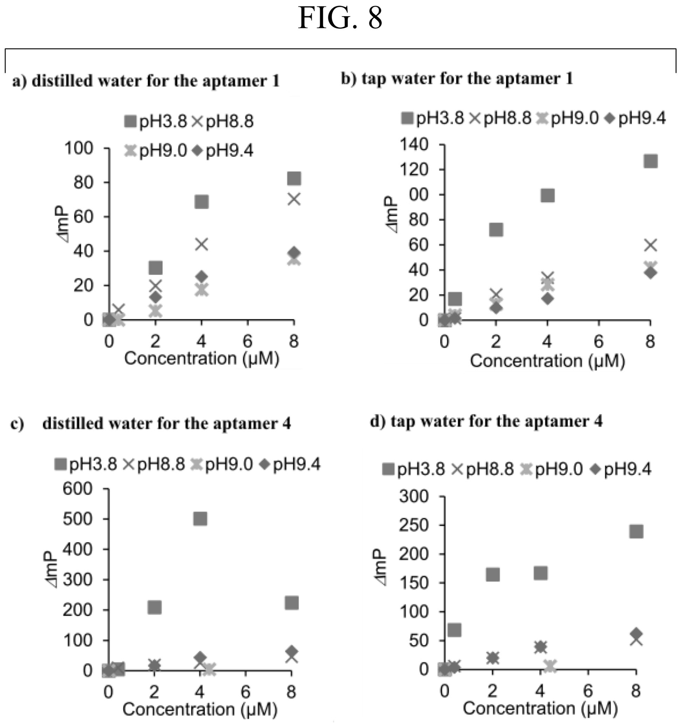

   Fig. 8 is a graph illustrating the relationship between the pH of the buffer used to dissolve the aptamer 1 or the aptamer 4, and the fluorescence polarization degree of the mixed solution of the buffer used for the dissolution and a cadmium solution.
[Fig. 9]

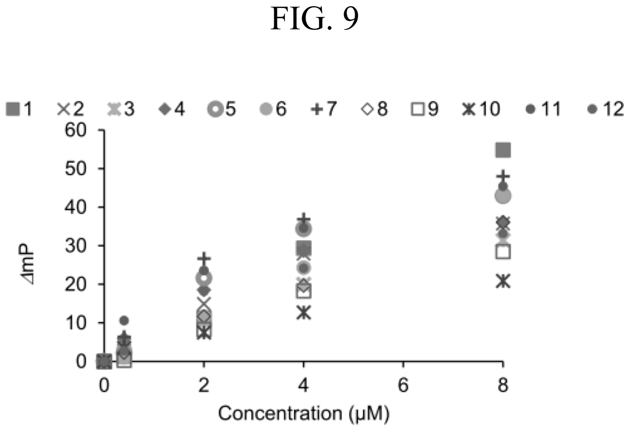

   Fig. 9 is a graph illustrating the relationship between the final cadmium concentration in solutions obtained by mixing, with a cadmium solution, the aptamers 1 to 12 dissolved in the respective buffer solutions, and the fluorescence polarization degree of the mixed solutions.
[Fig. 10]

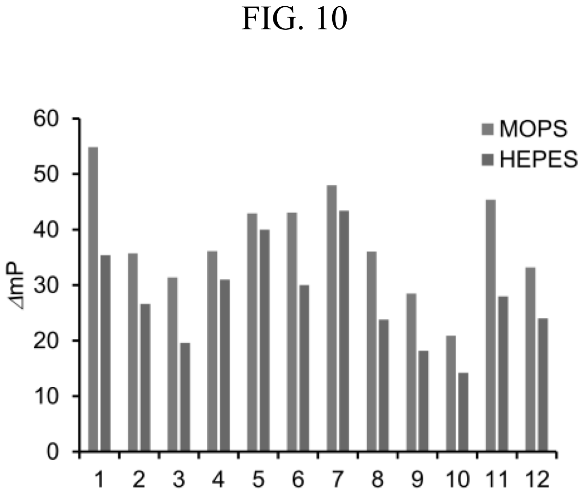

   Fig. 10 is a graph illustrating buffer dependence of the fluorescence polarization degree in the mixed solutions of the aptamers 1 to 12 dissolved in MOPS buffer or HEPES buffer, and a cadmium solution. For each aptamer, the left bar shows the result obtained using MOPS buffer, and the right bar shows the result obtained using HEPES buffer.
[Fig. 11]

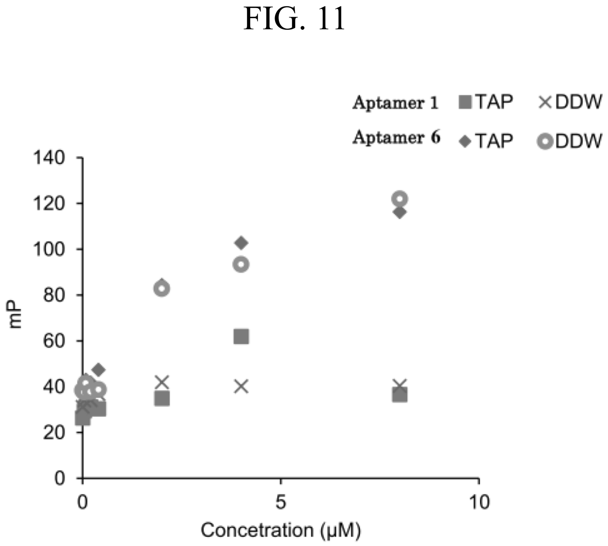

   Fig. 11 is a graph illustrating the relationship between the concentration of a cadmium-containing solution to be mixed using a buffer solution of each of the aptamer 1 and the aptamer 4, and the fluorescence polarization degree of a mixed solution of the aptamer and the cadmium solution in carbonate-bicarbonate buffer (pH 10).
[Fig. 12]

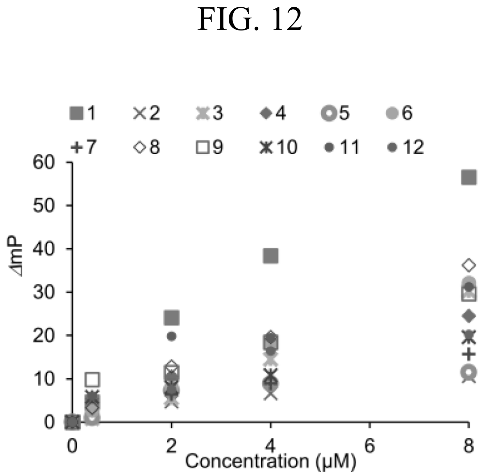

   Fig. 12 is a graph illustrating the relationship between the final cadmium concentration in a solution obtained by mixing each of the aptamer 1 to 12 solutions and a cadmium solution dissolved in well water (groundwater), and the fluorescence polarization degree of the mixed solution.
[Fig. 13]

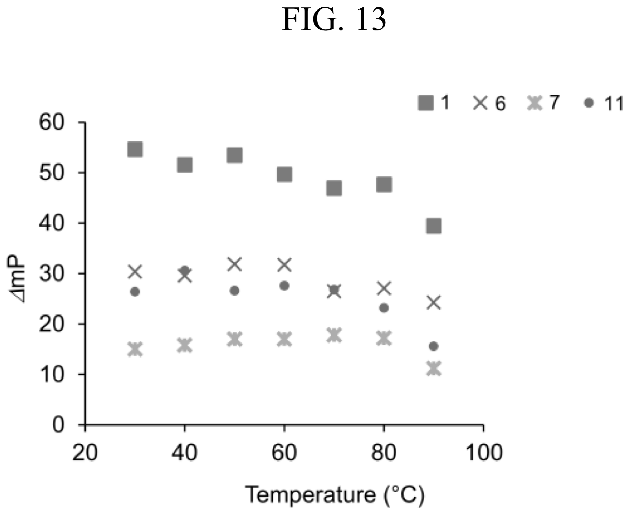

   Fig. 13 is a graph illustrating the relationship of the fluorescence polarization degree of a mixed solution obtained by mixing each of the aptamers 1, 6, 7, and 11 dissolved in MOPS buffer with a cadmium ion-containing solution, at various temperatures.
[Fig. 14A]

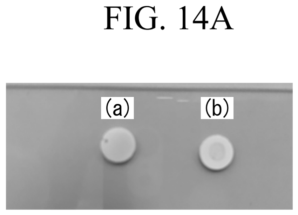

   Fig. 14A illustrates a photograph taken under indoor light of filters onto which a cadmium ion solution or a blank solution had been dropped, and then an aptamer solution of the present invention had been added. (a) corresponds to a filter onto which the blank solution not containing cadmium ions has been dropped, and (b) corresponds to a filter onto which a cadmium ion-containing solution (1 mM) has been dropped.
[Fig. 14B]

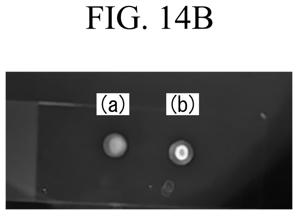

   Fig. 14B illustrates a photograph, taken under blue light through an orange filter, of the filters (a) and (b) taken in Fig. 14A.

### [Description of Embodiments]

Embodiments of a detection kit, a detection device, and a detection method for detecting a trace amount of a heavy metal in a sample of the present invention will now be described.

These embodiments provide a novel approach for rapid and highly sensitive detection of a heavy metal contained in water, soil, a food ingredient, a food product or the like, by utilizing change in fluorescence intensity or fluorescence polarization intensity of a fluorescent molecule (fluorophore) chemically bonded to an aptamer, caused when the aptamer binds to the heavy metal. Since a trace amount of the heavy metal contained in water, soil, a food ingredient, a food product or the like can be more rapidly detected, human exposure to the heavy metal can be reduced.

As one characteristic of the method for detecting a heavy metal of the present invention, the detection is performed by combining fluorescence polarization and an aptamer. The present inventors have made earnest studies on molecules working as sensors for heavy metals, and detection protocols for detecting heavy metals, with the aim of establishing a simple and rapid method for detection and quantification of heavy metals contained in environmental water, drinking water, soil, and the like. During these studies, the inventors have discovered that these heavy metals present in samples of environmental water, drinking water, soil and the like can be detected and measured by using, as sensor molecules, a fluorescently labeled aptamer which selectively binds to highly toxic heavy metals such as cadmium, mercury, and arsenic, in combination with an existing fluorescence polarization analyzer.

An aptamer is an artificially synthesized DNA, RNA, or peptide, or a derivative thereof, having a medium molecular size (10 to 100 nucleotides in the case of DNA or RNA), and is a molecule that can be made to selectively and specifically bind to a target substance by appropriately designing a sequence thereof. In the present embodiment, a DNA aptamer consisting of a single-stranded DNA modified with a fluorescent molecule is used as a probe for capturing a heavy metal. The DNA aptamer can be obtained in a relatively short period through search (selection) techniques represented by the SELEX method. Alternatively, the DNA aptamer can be mass-produced at a comparatively low cost through chemical synthesis.

Antibodies composed of proteins have been conventionally widely used as molecules that bind to target substances. The DNA aptamer has a molecular weight approximately 1/10 or less than that of an antibody, but exhibits high target selectivity equivalent to that of an antibody. A DNA aptamer consisting of a single-stranded DNA is generally a more stable compound than an antibody consisting of protein. For example, a dried DNA aptamer does not always require refrigeration or freezing for storage, and can be stored for a long period. This point is one of significant advantages over an antibody.

Referring to Fig. 1, an aptamer-based heavy metal detection method using fluorescence polarization degree measurement will be described. The fluorescence polarization analysis method is a method for analyzing the interaction between a fluorescent molecule (herein including a "molecule bonded to a fluorescent molecule") and a target substance by measuring a fluorescence polarization degree with a fluorescence polarimeter.

A fluorescence polarization degree refers to the extent to which a fluorescent molecule rotates after excitation until fluorescence emission. When a three-dimensional structure of the fluorescent molecule is formed, the rotational freedom of the fluorescent molecule decreases depending on the structure, and the rotation of the fluorescent molecule slows down. At this point, the fluorescence polarization degree increases. Thus, the movement of a fluorescent molecule is either reduced due to the influence of the surrounding three-dimensional structure, or is ceased, namely, immobilized through attachment to a large amount of a target substance. As a result, the rotation of the fluorescent molecule becomes strictly limited, and the fluorescence polarization increases. By measuring the fluorescence polarization degree, the interaction between the fluorescent molecule and the target substance can be rapidly analyzed, even when the amount of the sample is small.

Fig. 1A illustrates a conceptual diagram of a method for detecting a trace amount of a heavy metal through fluorescence polarization using a DNA aptamer of the present embodiment. A DNA aptamer used in the present embodiment has a fluorescent molecule bonded to at least one of the 3'-end and the 5'-end of a single-stranded DNA. The fluorescent molecule is generally bonded to the DNA aptamer via a linker. Hereinafter, a DNA aptamer modified with a fluorescent molecule is also referred to as a "fluorescently labeled DNA aptamer". A target substance of the DNA aptamer of the present embodiment is a heavy metal contained in environmental water, drinking water, soil, and the like.

In an environment where the target heavy metal is absent, a fluorescently labeled DNA aptamer 1 labeled with a fluorescent molecule 2 exists as a single strand. At this time, the fluorescent molecule 2 bonded to the DNA aptamer 1 rotates freely in a solution where the fluorescently labeled DNA aptamer 1 is dissolved. This corresponds to the state before heavy metal binding, from which an arrow is pointing in Fig. 1A. At this point, the fluorescence polarization degree of the solution is low.

When this fluorescently labeled DNA aptamer contacts with a heavy metal, that is, the target substance, in the solution, the three-dimensional structure thereof changes so that a certain part of the single strand forms a loop to surround the heavy metal. Meanwhile, a sequence portion of the DNA aptamer that is not directly involved in the binding to the heavy metal forms a double strand, resulting in an overall stem-loop structure (also called a hairpin structure). The DNA aptamer having this stem-loop structure forms a three-dimensional structure, and is stabilized as a whole. A part pointed by the arrow in Fig. 1A illustrates an image of a fluorescently labeled DNA aptamer 1' that has bound to a heavy metal 10 to form a stem-loop structure. The stem portion forming the double strand contributes to the stabilization of the three-dimensional structure after the binding of the target substance (the heavy metal in the present embodiment).

The DNA aptamer that has taken the heavy metal 10 in and formed a three-dimensional structure has become structurally hardened. Consequently, the rotational movement of the fluorescent molecule labeling the DNA aptamer is also restricted and reduced. As a result, the fluorescence polarization increases.

In fluorescence polarization analysis, the concentration of the fluorescent molecules in the solution is, in principle, not taken into consideration, and therefore, a change in the concentration of the fluorescent molecules causes only a slight influence on the difference in the polarization value. On the other hand, fluorescence polarization is affected by the sample matrix, and therefore, it should be noted that the chemical components contained in the sample to be measured needs to be confirmed in advance.

In the heavy metal detection method of the present embodiment, the extent of heavy metal binding to the fluorescently labeled DNA aptamer is detected by measuring the extent of a change in fluorescence polarization degree (ΔmP). Since the fluorescence polarization analysis has high detection sensitivity, it is possible to detect the change in fluorescence polarization degree in an extremely small amount of sample.

Therefore, by using the fluorescently labeled DNA aptamer of the present embodiment, it is possible to detect a heavy metal at the sub-nanomolar level. The inventors have studied fluorescently labeled DNA aptamers having various sequences, and have ultimately developed a fluorescently labeled DNA aptamer-based heavy metal detection kit for detecting various heavy metals.

In the method for detecting a heavy metal of the present embodiment, the extent of the change in the fluorescence polarization degree of a sample solution is detected for measurement. Therefore, there is no need to separate and purify a fluorescently labeled DNA aptamer bound to the heavy metal from a fluorescently labeled DNA aptamer not bound to the heavy metal. This allows for a reduction in the time required to prepare a sample solution used for the measurement, and hence the overall time required for the detection can be reduced. As a result, a sample of water or soil desired to be measured can be rapidly subjected to measurement and detection in or around the spot. Moreover, the heavy metal detection method of the present embodiment can be used not only for the detection of a heavy metal in water and soil, but also extended to detection of pesticides and the like in food ingredients and products.

Examples of the fluorescent molecule to be bonded to the DNA aptamer of the present embodiment include commonly used fluorescent molecules such as fluorescein, fluorescein isothiocyanate (FITC), Alexa Fluor (R) 488, Alexa Fluor (R) 514, Texas Red (TM), and Cy3 group. These fluorescent molecules can work as donor fluorescent molecules. Fig. 1A illustrates, as an example, a DNA aptamer using FITC group as the donor fluorescent molecule.

When the heavy metal binds to the fluorescent molecule bonded to the DNA aptamer, the three-dimensional structure of the aptamer changes as mentioned above, which also causes restriction in the rotation of the fluorescent molecule. This restriction of the rotation leads to an increase in the fluorescence polarization degree, and by measuring the extent of this increase in the fluorescence polarization degree, the presence or absence of binding to the heavy metal can be detected.

As illustrated in the example in Fig. 1A, in the present embodiment, the DNA aptamer to which only one fluorescent molecule is bonded can be used as a detection probe based on fluorescence polarization, but another fluorescent molecule that works as a FRET acceptor may be bonded to the DNA aptamer in addition to the fluorescent molecule working as a donor. An example of detection using such a fluorescently labeled DNA aptamer is illustrated in Fig. 1B. Examples of the acceptor fluorescent molecule include commonly used fluorescent molecules such as a 5-carboxy tetramethylrhodamine (TAMRA) group, Cy5 group, Cy5.5 group, ROX group, Alexa Fluor (R) 555, and Alexa Fluor (R) 647.

Similarly to the example described above, a fluorescent molecule that can work as a donor and a fluorescent molecule that can work as an acceptor are bonded to appropriate positions in a DNA aptamer of a single molecule. When a heavy metal, that is, the target substance, binds to this DNA aptamer, the three-dimensional structure is changed, and thus, a distance between the donor fluorescent molecule and the acceptor fluorescent molecule decreases.

In this state, when light of an excitation wavelength of the donor fluorescent molecule is irradiated, Fluorescence Resonance Energy Transfer (FRET) occurs between the two fluorescent molecules of the DNA aptamer of a single molecule, and energy is transferred to the acceptor fluorescent molecule due to the excitation of the donor fluorescent molecule. As a result, the acceptor fluorescent molecule exhibits fluorescence. Therefore, the presence or absence of the binding of the heavy metal to the fluorescently labeled DNA aptamer can be detected as a change in the fluorescence polarization degree or an increase in the fluorescence intensity at a detection wavelength of the acceptor fluorescent molecule.

In the example illustrated in Fig. 1B, to a single molecule of a DNA aptamer 11, a fluorescent molecule 12 working as a donor (FITC in this example) and another fluorescent molecule 13 working as an acceptor (TAMRA in this example) are bonded at appropriate positions on the DNA aptamer (specifically, one at each end of the DNA strand in this example). When a heavy metal, that is, the target substance, approaches the fluorescently labeled DNA aptamer to a sufficient distance, the three-dimensional structure of the aptamer 11 changes, causing the FITC and TAMRA to become closer in distance.

At this time, in a fluorescently labeled DNA aptamer 11' to which the heavy metal, the target substance, has bound, FRET is caused between the FITC molecule corresponding to the donor and the TAMRA molecule corresponding to the acceptor, resulting in an increase in the fluorescence intensity derived from the TAMRA molecule. In the example of Fig. 1B, by irradiating light at the excitation wavelength of FITC used as the donor fluorescent molecule 12, and measuring the change in the fluorescence intensity at the wavelength derived from the TAMRA molecule used as the acceptor fluorescent molecule 13, it is possible to detect whether or not the heavy metal is present in the measurement system.

It is also possible to use, as a fluorescence polarization detection probe, a DNA aptamer of the present embodiment to which only the aforementioned acceptor fluorescent molecule is bonded.

Furthermore, not only a fluorescent molecule but also a quencher molecule, which itself does not produce fluorescence but quenches the fluorescence derived from another molecule, may be additionally bonded to the DNA aptamer of the present embodiment. Examples of the quencher molecule include those commonly used such as DABCYL, Tide Quencher, QSY7, and Black Hole Quencher (BHQ).

An aptamer in which a fluorescent molecule working as the donor and a quencher molecule are bonded respectively via linkers to appropriate positions within a DNA aptamer of a single molecule is synthesized. Applying this to the example in Fig. 1B, the quencher molecule is bonded instead of the acceptor fluorescent molecule 13. When the target substance binds to this DNA aptamer, the three-dimensional structure changes, causing the fluorescent molecule and the quencher molecule to become closer in distance.

In this state, when light of the excitation wavelength of the donor fluorescent molecule is irradiated, the fluorescent molecule functions as the donor, and FRET is caused with the quencher molecule. As a result, the fluorescence intensity at the detection wavelength of the donor fluorescent molecule decreases. In this manner, the presence or absence of the binding of the heavy metal to the fluorescently labeled DNA aptamer can be detected as a decrease in the fluorescence intensity at the detection wavelength of the donor fluorescent molecule.

Thus, the fluorescently modified DNA aptamer used in the present embodiment can be a useful tool usable for the detection of heavy metal ions in a sample, as a system for detecting a change in fluorescence polarization degree, a system for detecting a change in fluorescence intensity, a system for detecting fluorescence ON-OFF, or a colorimetric detection tool, all driven by the structural change accompanying the binding to the target substance.

A DNA aptamer can be made into a detection probe utilizing fluorescence by chemically modifying any position within the sequence thereof with a fluorescent molecule. Such freedom in modification is another advantage of DNA aptamers over antibodies. Furthermore, a DNA aptamer can be designed to allow for a far greater change in three-dimensional structure and mobility upon binding to a target substance than is possible with antibodies.

In using a DNA aptamer that has both a donor fluorescent functional group and an acceptor fluorescent functional group bonded within a single molecule, the fluorescence wavelength derived from the acceptor fluorescent molecule can be used for the detection as described above. In this manner, the target substance can be detected in a short time without the need for a purification step, even when a fluorescently labeled DNA aptamer not bound to the target substance and a fluorescently labeled DNA aptamer bound to the target substance are present together in the measurement system.

Furthermore, in the detection systems illustrated in Fig. 1A and Fig. 1B, when FITC group is used as the donor fluorescent molecule, it is also possible to measure the extent to which the fluorescence intensity decreases by measuring the fluorescence wavelength derived from FITC group, instead of measuring the fluorescence polarization.

Although the examples of Figs. 1A and 1B illustrate an image in which only one heavy metal ion binds to the DNA aptamer, the number of heavy metal ions that bind to the DNA aptamer is not limited to one, and depending on the design of the DNA aptamer, a plurality of heavy metal ions may bind to the DNA aptamer depending on the concentration of the heavy metal ions within the measurement system. In the case where a plurality of heavy metal ions bind to the DNA aptamer, the three-dimensional structure is presumed to change even more significantly.

In the heavy metal detection method of the present embodiment, a buffer for a solution used in the measurement is preferably 3-(N-morpholino) propanesulfonic acid buffer (hereinafter referred to as MOPS buffer), 2-[4-(2-hydroxyethyl)-1-piperazinyl]ethanesulfonic acid buffer (hereinafter referred to as HEPES buffer), or carbonate-bicarbonate buffer. It is more preferable to use a carbonate-bicarbonate buffer with a pH of around 10.

Although details will be described later, when MOPS buffer was used in the detection system in Experimental Examples, an aminomethylfluorescein derivative (FAM)-labeled aptamer showed a higher fluorescence intensity than in using other buffer systems. Furthermore, the pH of the buffer affects the fluorescence polarization degree.

The present embodiment can provide a detection kit including the fluorescently labeled DNA aptamer described above. For example, the kit can be one including the fluorescently labeled DNA aptamer which has been powdered by, for example, freeze-drying, as a reagent, along with MOPS buffer, HEPES buffer, or carbonate-bicarbonate buffer. A microplate or a filter for the detection may also be included in the set if necessary.

In Experimental Examples described below, examples of the heavy metal detection using the fluorescently labeled DNA aptamer on a microplate and on a filter are described. As a tool used for the detection, a detection member capable of measuring fluorescence polarization can be used, in addition to a microplate and a filter. Such a detection member may also be included in the detection kit of the present embodiment.

Fig. 2 illustrates an example of the detection member. In a detection member 30 illustrated in Fig. 2, patches 41 to 45, which are obtained by impregnating with a test reagent of different concentrations and then drying the resultant, are attached to a paper-based substrate 31. As an example, five patches are attached, with the reagent concentration increasing by a factor of tenfold between each. A blank patch 50, which does not contain the test reagent, is also disposed on the substrate 31. The test member thus has a portable size so that the test can be conducted in the spot of a water resource or soil to be tested, such as a water source or a farmland. As the test reagent in this example, the fluorescently labeled DNA aptamer described above is used.

An example of a method for using the detection member is as follows: A test solution containing a sample, such as water or soil, that may contain a heavy metal, that is, a substance to be detected, is prepared, and is then dropped onto the patches of the respective concentrations. If the solution contains the substance to be detected, change in color of the patches, which is caused by FRET or quenching occurring in the fluorescently labeled DNA aptamer contained in the patch, is observed. When the change in color of the patch cannot be clearly observed, as illustrated in Figure 3, an LED light irradiation device 60 is used to subsidiarily irradiate the detection member 30 with LED light 61 of a prescribed wavelength (for example, 480 nm when the fluorescently labeled DNA aptamer contains FITC group), and the change in color is observed. The LED light irradiation device 60 can be one that is of a portable size.

By using such a detection member, it is possible to detect the presence or absence of a heavy metal more simply and rapidly in or around the spot where a test subject, such as water, soil, a food ingredient, or a food product, is present, for example, in a food processing plant, a water source, a construction site, or a farmland.

If the substance to be tested cannot be simply detected using the aforementioned detection member and the portable LED light irradiation device, fluorescence polarization degree measurement is performed on the detection member to measure the concentration of the substance to be tested. For the fluorescence polarization degree measurement, a calibration curve for the substance to be tested is prepared in advance for each concentration of the test reagent impregnated into the patches.

In the present embodiment, a generally used fluorescence spectrophotometer can be used as a fluorescence detection device, and a fluorescence polarization analyzer can be used as a fluorescence detection device.

Fig. 4 illustrates an example of the fluorescence polarization detection device used for the fluorescence polarization degree measurement. The detection member 30 (containing the fluorescently labeled DNA aptamer having FITC group) is irradiated with light having a wavelength of 480 nm by a fluorescence polarization detection device 70. An image during the irradiation is captured by a camera equipped with a sensor, and the resultant image data is then analyzed using analysis software to quantitatively determine the concentration of the substance to be tested.

Although Fig. 4 illustrates a detection example using the detection member 30 of Fig. 2, it is also possible to quantitatively detect the substance to be detected in a sample having been dropped onto another member, such as a microplate or a filter, using the same type of fluorescence polarization detection device.

In this manner, by using, as a detection kit, a fluorescently labeled DNA aptamer designed and created according to a substance to be detected, the presence or absence and the concentration of the substance to be detected such as a heavy metal can be analyzed rapidly, simply, and at a low cost.

When the detection method of the present embodiment is employed, the presence or absence of a heavy metal can be confirmed rapidly in the spot where a test subject, such as water or soil, is present. Furthermore, if necessary, the content of the heavy metal can be quantitatively determined. Accordingly, compared to a conventional method, heavy metal detection can be performed simply and at a low cost without the need for highly advanced technology.

In Experimental Examples described below, cadmium is described as a heavy metal corresponding to the target substance, but the heavy metal detectable in the present embodiment is not limited to cadmium. Mercury and arsenic can also be used as target substances in the detection method of the present embodiment.

### Examples

The following reagents were used in Experimental Examples described herein. A stock solution of 1000 µM cadmium ions was freshly prepared, before each experiment, using Cadmium Chloride 2.5-hydrate (FUJIFILM Wako Pure Chemical Corporation, Japan). A fluorescently labeled DNA aptamer was synthesized by FASMAC Co., Ltd. (Japan). Fluorescence polarization assay and fluorescence intensity measurement were performed and recorded using a TECAN Spark 10, with an excitation wavelength of 480 nm and an emission wavelength of 520 nm.

### [Experimental Example 1] Confirmation of Sequence Dependence of Fluorescently Labeled DNA Aptamer in Cadmium Ion Detection

The sequence dependence of the ability to bind to a heavy metal was confirmed by using fluorescently labeled DNA aptamers having various sequences. The sequences of the aptamers used in Experimental Examples are summarized in Table 1. All of the aptamers listed in Table 1 possess 32 to 38 nucleotides, and FAM group bonded at either the 5'-end or the 3'-end. The aptamers having sequences set forth in SEQ ID NOs: 1 to 12 are hereinafter referred to as aptamer 1 to aptamer 12, respectively.

**[Table 1]**

| SEQ ID NO: | Sequence (5' to 3') |
|---|---|
| **1** | GGACTGTTGTGGTATTATTTTTGGTTGTGCAGTCC-FAM |
| **2** | FAM-GGACTGTTGTTGTATTATTTTTGTTGTGCAGTCC |
| **3** | TTTTTGGACTGTTGTGCTATTATTTTTGCTAGTGC-FAM |
| **4** | TTTTTGGACTGTTGTGCTATTATTTTTGCTAGTGCAAA-FAM |
| **5** | FAM-GGACTGTTGTGGTATTATTTTTGGTTGTGCAGTCC-Biotin |
| **6** | FAM-GGACTGTTGTGGTATTATTTTTGGTTGTGCAGTCC-TAMRA |
| **7** | 5-Biotin -TTTTTGGACTGTTGTGCTATTATTTTTGCTAGTGCAAA-FAM |
| **8** | 5-Biotin-TTTTTGGACTGTTGTGCTATTATTTTTGCTAGTGC-FAM |
| **10** | 5-Biotin TTTTTGGACTGTTGTGGTATTATTTTTGGTTGTGC-FAM |
| **11** | CTCAGGACGACGGGTTCACAGTCCGTTGTC-FAM |
| **12** | FAM-CTCAGGACGACGGGTTCACAGTCCGTTGTC |

The aptamer 1 is a known aptamer of Reference¹, and has FAM group bonded to the 3'-end. The result obtained using the aptamer 1 corresponds to Comparative Example in Experimental Example 1. The aptamer 2 has the same sequence as SEQ ID NO: 1 in the nucleotide sequence portion, but has FAM group bonded to the 5'-end not to the 3'-end. The aptamer 3 is a known aptamer of Reference², and has a sequence obtained by substituting three nucleotides in total with different nucleotides, and adding five nucleotides on the 5'-end side and deleting five nucleotides at the 3'-end in the sequence of SEQ ID NO: 1. The aptamer 4 has a sequence obtained by further adding three nucleotides to the 3'-end in the sequence of SEQ ID NO: 3.

The aptamer 5 has the same sequence as SEQ ID NO: 1 in the nucleotide sequence portion, but has FAM group bonded to the 5'-end, and a 5-biotin group bonded to the 3'-end. The aptamer 6 has the same sequence as SEQ ID NO: 1 in the nucleotide sequence portion, but has FAM group bonded to the 5'-end, and TAMRA group bonded to the 3'-end.

The aptamers 7 and 8 have sequences obtained by respectively binding a 5-biotin group to the 5'-end in the sequences set forth in SEQ ID NOs: 4 and 3. The aptamer 9 has a sequence obtained by substituting five nucleotides on the 3'-end side with different nucleotides, and binding a biotin group thereto in the sequence of SEQ ID NO: 2. The aptamer 10 has a sequence obtained by substituting three nucleotides in total with different nucleotides in the sequence set forth in SEQ ID NO: 9.

The aptamers 11 and 12 are identical in the base portion, and have nucleotide sequences different from those of the aptamers 1 to 10. SEQ ID NOs: 11 and 12 differ only as to whether FAM group is bonded to the 3'-end (SEQ ID NO: 11) or the 5'-end (SEQ ID NO: 12).

### [Experimental Example 2] Relationship between Fluorescence Intensity of Aptamer and Buffer

10 µL of each of solutions of the 12 aptamers shown in Table 1 (0.5 µM) was dissolved in 230 µL of 10 mM 3-(N-morpholino) propanesulfonic acid buffer (hereinafter referred to as MOPS buffer) that is alkaline (pH 9.0), and fluorescence intensity derived from FAM group bonded to the aptamer was then measured. Similarly, 10 µL of each of the solutions of the 12 aptamers shown in Table 1 (0.5 µM) was dissolved in 230 µL of 10 mM 2-[4-(2-hydroxyethyl)-1-piperazinyl]ethanesulfonic acid buffer (hereinafter referred to as HEPES buffer), and fluorescence intensity derived from FAM group bonded to the aptamer was then measured.

Fig. 5 illustrates a graph comparing the fluorescence intensities of the aptamers 1 to 12 dissolved in MOPS buffer and HEPES buffer. It was found that, depending on the nucleotide sequence, a large difference is caused in the fluorescence intensity between the two types of buffers. In other words, it is presumed that the interaction between MOPS and FAM may differ significantly depending on the nucleotide sequence.

### [Relationship between Cadmium Ion Concentration and Fluorescence Intensity]

Subsequently, the aptamers 1 to 12 were used to conduct an experiment to confirm the relationship between the concentration of a solution containing cadmium to be mixed and the fluorescence intensity of the resultant mixed solution. 10 µL of each of the aptamers 1 to 12 (0.5 µM) was dissolved in 230 µL of either 10 mM MOPS buffer or HEPES buffer. To the resultant solution, a cadmium chloride solution with a prescribed concentration, dissolved in double-distilled water (hereinafter referred to as DDW), was added and mixed. After leaving the resultant for 20 minutes at room temperature, the fluorescence intensity was measured at 515 nm.

The results are illustrated in Fig. 6. The vertical axis indicates the relative fluorescence intensity, and the horizontal axis indicates the final concentration of cadmium ions. For each aptamer, assuming that the fluorescence intensity obtained when the cadmium ion concentration was 0 µM was 1, the change in fluorescence intensity depending on the concentration of cadmium ions added was defined as the relative fluorescence intensity. The aptamer 5 showed an increase in the fluorescence intensity as the cadmium ion concentration increased, but all the other aptamers showed a decrease of 10% or more, and particularly, the aptamer 1 showed a decrease of 60% or more, and the aptamer 6 showed a decrease of 90% or more. In the aptamer 1 and the aptamer 6, it is presumed that the three-dimensional structure of the aptamer changed through the binding of the cadmium ions to the aptamer, and hence the fluorescence intensity derived from FAM group was affected.

Moreover, in order to confirm the buffer dependence of the aptamers 1, 5, and 6, each of the aptamers 1, 5, and 6 was dissolved in 230 µL of 10 mM MOPS buffer or HEPES buffer. To the resultant solution, a cadmium chloride solution with a prescribed concentration, dissolved in DDW, was added and mixed. After leaving the resultant for 20 minutes at room temperature, the fluorescence intensity was measured at 515 nm. The results are illustrated in Fig. 7. The vertical axis indicates the fluorescence intensity, and the horizontal axis indicates the number of the aptamer. The fluorescence intensity of Fig. 7 shows a relative value obtained by assuming that the fluorescence intensity of an aptamer solution in which no cadmium was added was 1. Although the difference between buffers was small for the aptamer 1 and the aptamer 5, the aptamer 6 showed a difference in the fluorescence intensity of more than two-fold due to the difference in buffer used.

### [Relationship between pH of Buffer and Fluorescence Polarization Degree]

An experiment was conducted using the aptamer 1 and the aptamer 4. 10 µL of either the aptamer 1 or the aptamer 4 (0.5 µM) was dissolved in 230 µL of 10 mM MOPS buffer, to confirm the influence of the pH of a buffer used on the fluorescence polarization degree. To the resultant solution, a cadmium chloride solution with a predetermined concentration, dissolved in either DDW or well water, was added and mixed. After leaving the resultant for 20 minutes at room temperature, the fluorescence intensity was measured at 515 nm. The well water (groundwater) used was collected from a well or a tap near the experimental location of the inventors.

The results are illustrated in Fig. 8. The vertical axis indicates the fluorescence polarization degree (ΔmP value), and the horizontal axis indicates the final concentration of cadmium ions. In Fig. 8, (a) shows the results using the aptamer 1 and a cadmium chloride solution in DDW, (b) shows the results using the aptamer 1 and a cadmium chloride solution in well water, (c) shows the results using the aptamer 4 and a cadmium chloride solution in DDW, and (d) shows the results using the aptamer 4 and a cadmium chloride solution in well water. In the pH comparison results obtained using the aptamer 1, the highest ΔmP value was exhibited at an acidic pH of 3.8. Similarly, in the pH comparison results obtained using the aptamer 4, the highest ΔmP value was also observed at an acidic pH of 3.8. In the results obtained using the aptamer 1, on the alkaline side, the fluorescence polarization degree tends to decrease, due to the influence of salts contained in the well water, compared to the sample using distilled water, but on the acidic side, the influence of the salts is not very noticeable. For the aptamer 4, the polarization degree generally tends to decrease generally in the well water sample.

### [Experimental Example 3] Relationship between Cadmium Concentration and Fluorescence Polarization Degree

An experiment was conducted using the aptamers 1 to 12, to confirm the relationship between the concentration of a solution containing cadmium to be mixed and the fluorescence polarization degree of the resultant mixed solution. To a solution obtained by dissolving 10 µL of each of the aptamers 1 to 12 (0.5 µM) in 230 µL of 10 mM MOPS buffer, a cadmium chloride solution with a predetermined concentration, dissolved in DDW, was added and mixed. After leaving the resultant for 20 minutes at room temperature, the fluorescence polarization degree was measured at 515 nm. The fluorescence polarization degree was measured with Spark manufactured by TECAN.

The results are illustrated in Fig. 9. The vertical axis indicates the fluorescence polarization degree, and the horizontal axis indicates the final concentration of cadmium ions. In using all the aptamers, the fluorescence polarization degree increased as the cadmium concentration increased. It is presumed that the increase in the fluorescence polarization degree (ΔmP) is due to the structural change of the aptamer, as expected, caused by the restriction of the free rotation of FAM group and the increase in molecular weight associated with the binding of cadmium in a large amount to the aptamer, that is, an oligonucleotide.

Moreover, in order to confirm the buffer dependence of the fluorescence polarization degree of the mixed solution of any of the aptamers 1 to 12 and the cadmium solution, to a solution obtained by dissolving each of the aptamers 1, 5, and 6 in 230 µL of 10 mM MOPS buffer, a cadmium chloride solution with a predetermined concentration, dissolved in DDW, was added and mixed, and the fluorescence polarization degree of the resultant mixed solution was measured. The results are illustrated in Fig. 10. It is presumed that these results were obtained due to the polarization effect of the aptamer enhanced by the interaction between the MOPS molecule and FAM group. All aptamers exhibited higher ΔmP values in using MOPS buffer than in using HEPES buffer.

Moreover, an experiment was conducted using a carbonate-bicarbonate buffer solution at pH 10 of each of the aptamer 1 and the aptamer 6, to confirm the relationship between the concentration of a solution containing cadmium to be mixed and the fluorescence polarization degree. 10 µL of the aptamer 1 or the aptamer 6 (0.5 µM) was dissolved in 230 µL of 10 mM carbonate-bicarbonate buffer. To the resultant solution, a cadmium chloride solution with a predetermined concentration, dissolved in DDW or tap water, was added and mixed. After leaving the resultant for 20 minutes at room temperature, the fluorescence polarization degree was measured at 515 nm.

The results are illustrated in Fig. 11. In the aptamer 1, no increase or decrease in the fluorescence polarization degree was observed in using DDW even when the cadmium concentration was varied, and a slight fluctuation was observed in using tap water. In the aptamer 4, the fluorescence polarization degree increased as the cadmium concentration increased in using both DDW and tap water.

### [Experimental Example 4] Influence on Fluorescence Polarization of Cadmium Solution in Well Water

In actual farms and construction sites, a heavy metal is dissolved in water that contains other metals and the salts thereof, rather than being dissolved in distilled water. In order to confirm the influence of such an environment on the fluorescence polarization degree of a sample, a sample was prepared by dissolving cadmium chloride in well water instead of DDW, the resultant was subjected to the aptamer binding, and the fluorescence polarization degree was measured. The well water (groundwater) used was collected from a well or a tap near the experimental location of the inventors. The electrical conductivity of the well water used was 0.3 mS/cm, which was higher than the electrical conductivity of general Japanese river water (0.06 mS/cm). The experiment was conducted under the same conditions as in Experimental Example 3, except that the well water was used instead of DDW for preparing the cadmium chloride solution.

The results are illustrated in Fig. 12. The vertical axis indicates the fluorescence polarization degree, and the horizontal axis indicates the final concentration of cadmium ions. In all the aptamers, the fluorescence polarization degree increased as the cadmium concentration increased. Only the aptamer 1 showed a higher fluorescence polarization degree compared to that obtained in the DDW solution, while all the other aptamers exhibited a lower fluorescence polarization degree than in the DDW solution. Furthermore, the types of aptamers showing a high fluorescence polarization degree also exhibited a trend different from that obtained in the DDW solution. These results suggest that binding of metals other than cadmium and the salts thereof contained in the well water to the aptamer caused a change in the structure of the aptamer, affecting the behavior of FAM group.

### [Experimental Example 5] Relationship between Temperature and Fluorescence Polarization Degree

A confirmation was made regarding the influence of various temperatures on the fluorescence polarization degree in mixed solutions of MOPS buffer solutions of the aptamers 1, 6, 7, and 11 with the cadmium ion-containing solution. After leaving the mixed solutions at a prescribed temperature for 30 minutes, the respective fluorescence polarization degrees were measured.

The results are illustrated in Fig. 13. The vertical axis indicates the ΔmP value, and the horizontal axis indicates the temperature. In the aptamers 6, 7, and 11, no significant difference in the fluorescence polarization degree was observed, and stable values were obtained in the range of 30°C to 60°C, but a decrease in the fluorescence polarization degree was observed above 70°C. In contrast, in the aptamer 1, the fluorescence polarization degree tended to show a decrease across the range from 30°C to 90°C. It was confirmed that when the aptamers employed in this example were used, the detection could be appropriately performed through the measurement at room temperature. In other words, by using the aptamers of this example, the presence or absence of a heavy metal can be simply detected in the spot such as a water source, a farmland, or a food processing plant without the need for large-scale equipment.

### [Experimental Example 6] Detection of Cadmium Ions on Filter

While the detection experiment was performed in a microplate in Experimental Example 1, heavy metal detection was attempted on a filter in Experimental Example 6.

The aptamer 6 having the sequence set forth in SEQ ID NO: 6 shown in Table 1 was used as the aptamer.

5 µL of the aptamer (1 µM) was added to a membrane filter and air-dried. After the drying, 1 µL of MOPS buffer solution containing cadmium was added to the filter impregnated with the aptamer. After the addition, the filter was irradiated with a blue LED and observed through an orange filter.

Fig. 14A illustrates a photograph taken under indoor light of filters onto which the cadmium ion solution or a blank solution had been dropped, and then the aptamer solution of the present embodiment had been added. A filter (b) on the right side corresponds to a filter onto which the cadmium ion-containing solution (1 mM) had been dropped, and a filter (a) on the left side corresponds to a filter onto which the blank solution not containing cadmium ions had been dropped.

Fig. 14B illustrates a photograph, taken under blue light through an orange filter, of the filters (a) and (b) taken in Fig. 14A. Compared to the filter (a), light with a wavelength near 560 nm was strongly detected in the filter (b). This wavelength is that of the fluorescence derived from TAMRA group contained in the aptamer 6. From this result, it was confirmed that while no structural change occurred in the aptamer on the filter (a), onto which the blank solution had been dropped, FRET occurred on the filter (b), onto which the cadmium ion-containing solution had been dropped, due to a change in the three-dimensional structure of the aptamer, namely, it was confirmed that the cadmium ions were trapped by the aptamer.

In this manner, it was found that the presence of cadmium ions contained in an aqueous solution can be detected by using the fluorescence polarization detection kit of Experimental Example.

All Experimental Examples described above used cadmium as the heavy metal for measurement. As a result, it was revealed that the presence or absence of cadmium in a sample, and concentration thereof can be detected by the method of Experimental Example. The method of Experimental Example allows for simple and rapid detection of cadmium in a sample in a short time, compared to conventional methods, without requiring advanced analytical skills.

A properly designed aptamer binds selectively to a target heavy metal. Therefore, by pre-selecting and preparing aptamers that can selectively bind to other heavy metals, it is expected that these other heavy metals can also be detected simply in a short time by a method similar to the method of Experimental Example. Moreover, it is not only a heavy metal that the aptamer selectively binds, but an aptamer can also be designed to bind to a specific compound. Therefore, the aptamer and the detection method of the present embodiment are not limited to use for the detection of a heavy metal but can be used also for detecting, for example, pesticides or impurities in food ingredients and food products.

### [References]

1. Liu Y, Zhang D, Ding J, Hayat K, Yang X, Zhan X, Zhang D, Lu Y, Zhou P (2021) A Facile Aptasensor for Instantaneous Determination of Cadmium Ions Based on Fluorescence Amplification Effect of MOPS on FAM-Labeled Aptamer. Biosensors 11::133
2. Pavadai R, Perumal P (2022) Versatile sensing platform of an innovative copper oxide-assisted Cu-phenolic coordination nanosheet-mediated fluorophore-tagged GT-rich SSA-based fluorescence ON-OFF biosensor for the subsequent detection of Cd2+ and S2-ions. New Journal of Chemistry 46::3431-3447.

### [Reference Signs List]

- 1: fluorescently labeled DNA aptamer
- 1': fluorescently labeled DNA aptamer forming stem-loop structure
- 2: fluorescent molecule
- 10: heavy metal
- 11: fluorescently labeled DNA aptamer
- 12: donor fluorescent molecule
- 13: acceptor fluorescent molecule
- 30: detection member
- 31: substrate
- 41, 42, 43, 44, 45: patch
- 50: blank patch
- 60: LED light irradiation device
- 61: LED light
- 70: fluorescence polarization detection device

## Claims

1. A detection kit for detecting a trace amount of a heavy metal in a sample, comprising a DNA aptamer that is labeled with a fluorescent molecule, and selectively binds to a specific heavy metal.

2. The detection kit according to claim 1, wherein the heavy metal is at least one selected from the group consisting of cadmium, mercury, and arsenic.

3. The detection kit according to claim 1, wherein detection is performed by visual observation, fluorescence analysis, or fluorescence polarization analysis.

4. The detection kit according to claim 1, wherein the sample is water, soil, a food ingredient, or a food product.

5. The detection kit according to claim 1, wherein the DNA aptamer is labeled with two fluorescent molecules.

6. The detection kit according to claim 1, further comprising MOPS buffer, HEPES buffer, or a carbonate-bicarbonate buffer.

7. A detection device comprising:
a DNA aptamer that is labeled with a fluorescent molecule, and selectively binds to a specific heavy metal; and
a fluorescence measurement unit for measuring fluorescence intensity of the fluorescent molecule of the DNA aptamer,
wherein a trace amount of the heavy metal in a sample is detected by measuring fluorescence intensity or fluorescence polarization intensity derived from the fluorescent molecule labeling the DNA aptamer bound to the specific heavy metal.

8. The detection device according to claim 7, wherein the heavy metal is at least one selected from the group consisting of cadmium, mercury, and arsenic.

9. The detection device according to claim 7, wherein detection of the heavy metal is performed by fluorescence analysis or fluorescence polarization analysis.

10. The detection device according to claim 7, wherein the sample is water, soil, a food ingredient, or a food product.

11. A detection method for detecting a trace amount of a heavy metal in a sample, comprising the steps of:
obtaining a mixed solution by mixing a solution, in which a DNA aptamer labeled with a fluorescent molecule and specifically binding to the specific heavy metal is dissolved, with a liquid containing a sample potentially containing the specific heavy metal;
measuring fluorescence intensity or fluorescence polarization intensity derived from the fluorescent molecule of the DNA aptamer contained in the mixed solution; and
detecting the specific heavy metal based on a result of the measurement.

12. The detection method according to claim 11, wherein the step of measuring fluorescence polarization intensity further comprises a step of measuring a concentration of the heavy metal.
